# EUROPEAN PATENT APPLICATION

(11) **EP 1 908 511 A1**
(43) Date of publication of application: **09.04.2008**
(21) Application number: 06729843.0
(22) Date of filing: 17.03.2006
(51) Int. Cl.: B01J 13/00, A61K 9/107, A61K 47/34, A61K 47/44, C08J 3/03, B01F 3/08

(54) **GRANULE DISPERSION COMPOSITION, PROCESS FOR PRODUCING THE SAME, AND GRANULAR MATERIAL AND MEDICINE**

(30) Priority: 29.06.2005 JP 2005190350
(71) Applicant: Mitsubishi Chemical Corporation, Minato-ku Tokyo 108-0014 (JP)
(72) Inventor: ISOJIMA, Tatsushi Mitsubishi Chem. Group Science &, Aoba-ku, Yokohama-shi, Kanagawa 227-8502 (JP); ASATANI, Haruki Mitsubishi Chem. Group Science &, Aoba-cho, Yokohama-shi, Kanagawa277-8502 (JP); SEKI, Hiroya, Aoba-ku, Yokohama-shi, Kanagawa 277-0038 (JP); TAKEUCHI, Hisao Mitsubishi Chem. Group Science &, Aoba-ku, Yokohama-shi, Kanagawa 277-8502 (JP); SEKI, Hiroya, Kanagawa 227-0038 (JP)
(74) Representative: Siegert, Georg
(86) International application number: PCT/JP2006/305895
(87) International publication number: WO 2007/000842

(57) **Abstract**

The subject matter of the present invention is to provide a new particulate material dispersion composite in which a material poorly soluble in water is micronized and dispersed. For the above purpose, a particulate material dispersion composite is produced in the present invention, wherein a particulate material containing a specific material which is poorly soluble in water, a polymer and a lipid is dispersed in water, the mean diameter of said particulate material is 1 µm or less, and the ratio of the combined weight of said polymer and said lipid to the weight of said specific material is 1.5 or larger.

## Description

### Technical Field

The present invention relates to a particulate material dispersion composite in which a particulate material containing a poorly water-soluble substance is dispersed in water, a method for producing the same, a particulate material used for said composite and a drug product using said composite.

### Background Art

In various fields of industry and medicine, a composite is used in which a poorly water-soluble compound is dispersed. For example, a compound poorly soluble in water is micronized and dispersed in water. In this way, drugs difficult to dissolve in water may be used for medical practice.

When poorly water-soluble drugs are administered to man, conventionally in many cases, the drugs have been administered as dispersion in water, as described above. As poorly water-soluble drugs are not easily absorbed into systemic circulation after administration, it takes a long time until the drug exhibits its effect, or the drug may be excreted from the body before it is absorbed into systemic circulation. The effect of the drug is then not satisfactory. Recently, therefore, in the area of drug formulation, micronization of drugs has been extensively studied as a means of drug delivery of poorly water-soluble drugs for practical use. By micronizing drugs, stability of the dispersion of the drugs in water will increase, leading to increased absorption into the body and enhanced effectiveness.

The method of micronization of this kind of poorly water-soluble drugs has been investigated in various ways. The methods investigated so far include, for example: mechanically crushing a large solid of poorly water-soluble drug using surface-ameliorating agents such as surfactant; injecting an organic solvent in which hardly water-soluble drug is dissolved, using a spray; dissolving a poorly water-soluble drug in a water-soluble organic resolvent and spreading it in water allowing precipitation with antisolvent; dissolving a poorly water-soluble drug in a water-insoluble organic resolvent, forming an emulsion with the aid of surfactant for example, and removing the water-insoluble organic resolvent (in-liquid drying method) (Patent Documents 1 to 8).
[Patent Document 1] US Patent No. 5145684
[Patent Document 2] Japanese Patent Laid-Open Publication No. Sho 63-232840
[Patent Document 3] Japanese Patent Laid-Open Publication No. Sho 57-27128
[Patent Document 4] Japanese Patent Laid-Open Publication No. Sho 63-122620
[Patent Document 5] Japanese Patent No. 3244502 [Patent Document 6] Japanese Patent Laid-Open Publication No. Hei 1-156912
[Patent Document 7] Published Japanese Patent translation of a PCT application No. Sho 61-63613
[Patent Document 8] Published Japanese Patent translation of a PCT application No. Hei 4-46115

### Disclosure of the Invention

### Problem to Be Solved by the Invention

When poorly water-soluble substances are dispersed in water as microparticles, further improvement in performance has been desired in various industry fields, depending on the specific use of the substance, and new technology has been sought to meet the requirements for the improvement. Take, for example, the above-mentioned drugs with poor water-solubility. By the previously known methods, a large amount of energy was required in micronization, the diameter of micronized drug particles was still not small enough, or the stability of drug formulation in water was not sufficient even if the diameter was small enough, and therefore there was room for further improvement. Therefore, in the medical field for example, a method has been required which can process poorly water-soluble drugs into a form which has sufficiently small particle diameter and yet stable, in an inexpensive way.

The present invention has been made to solve the above problem. Namely, the purpose of the present invention is to provide a new particulate material dispersion composite in which a substance poorly water-soluble is dispersed in water as micronized particles, a method for producing the composite, a particulate material to be used for the composite, and a drug product using the composite.

### Means for Solving the Problem

The present inventors made an intensive investigation to solve the above problems and found that, by removing a non-miscible solvent which is non-miscible with water from emulsion in which liquid droplets containing the non-miscible solvent, a specific material, a polymer and a lipid are dispersed in water, it is possible to obtain a particulate material dispersion composite, wherein a particulate material containing the specific material, the polymer and the lipid is dispersed in water, and the mean diameter of the particulate material is 1 µm or less. These findings led to the completion of the present invention.

Namely, the subject matter of the present invention lies in a particulate material dispersion composite, wherein a particulate material containing a specific material which is poorly soluble in water, a polymer and a lipid is dispersed in water, the mean diameter of said particulate material is 1 µm or less, and the ratio of the combined weight of said polymer and said lipid to the weight of said specific material is 1.5 or larger (claim 1). Through the use of the above composite, it is possible to disperse the particulate material in a medium in a hitherto unknown new mode.

Another subject matter of the present invention lies in a particulate material dispersion composite, wherein a particulate material contains a specific material which is poorly soluble in water, a polymer and a lipid are dispersed in water, the mean diameter of said particulate material is 1 µm or less, and said particulate material is stable even when left for 12 hours under the conditions of 25 °C and 1013 hPa (claim 2). Through the use of the above composite also, it is possible to disperse the particulate material in a medium in a hitherto unknown new mode.

Still another subject matter of the present invention lies in a particulate material containing a specific material poorly soluble in water, a polymer and a lipid, wherein the mean diameter of said particulate material is 1 µm or less, and the ratio of the combined weight of said polymer and said lipid to the weight of said specific material is 1.5 or larger (claim 13). Through the use of the particulate material, it is possible to provide a particulate material with a hitherto unknown new mode.

It is preferable that said specific material contains a drug (claim 8).

It is also preferable that said particulate material has a surfactant on its surface (claim 3).

Further, it is preferable that said polymer is poorly soluble in water (claim 4).

Further, it is preferable that said specific material is poorly soluble in said lipid (claim 5).

Further, it is preferable that said lipid and said polymer are phase-separated from each other (claim 6).

Further, it is preferable that said specific material, said polymer and said lipid are all soluble in at least one kind of non-miscible solvent which is non-miscible with water (claim 7).

Another subject matter of the present invention lies in a drug product containing the particulate material dispersion composite as defined above (claim 14). Through the use of this drug product, it will be easier to administer the particulate material of the drug product to treatment targets appropriately.

Still another subject matter of the present invention lies in a method for producing the particulate material dispersion composite, comprising the step of: removing a non-miscible solvent, which is non-miscible with water, from emulsion in which liquid droplets are dispersed in water, said liquid droplets containing said non-miscible solvent, said specific material, said polymer and said lipid (claim 9). By this method, it is possible to produce the above particulate material dispersion composite easily.

Still another subject matter of the present invention lies in a method for producing a particulate material dispersion composite, comprising the steps of: removing a non-miscible solvent, which is non-miscible with water, from emulsion in which liquid droplets are dispersed in water, said liquid droplets containing said non-miscible solvent, a specific material, a polymer and a lipid, and removing particles having a diameter larger than a predetermined value from the water (claim 10). By this method also, it is possible to produce the above particulate material easily.

Further, still another subject matter of the present invention lies in a method for producing a particulate material dispersion composite, comprising the step of: removing a non-miscible solvent, which is non-miscible with water, from emulsion in which liquid droplets are dispersed in water, said liquid droplets containing said non-miscible solvent, a specific material, a polymer and a lipid and having 1.5 or larger value of the ratio of the combined weight of said polymer and said lipid to the weight of said specific material (claim 11). By this method also, it is possible to produce the above particulate material easily.

It is preferable that the above method for producing the particulate material dispersion composite further comprises the step of: micronizing said liquid droplets in said emulsion. (claim 12).

### Advantageous effect of the invention

According to the particulate material dispersion composite of the present invention, it is possible to disperse in water a particulate material containing a poorly water-soluble specific material in a hitherto unknown new mode.

Further, according to the method for production of the particulate material dispersion composite of the present invention, the above particulate material can be produced easily.

Furthermore, the particulate material of the present invention provides a particulate material of a hitherto unknown new mode.

Furthermore, the drug product of the present invention makes possible easy administration of particulate material of drugs to treatment targets in an appropriate manner.

### Brief Description of the Drawings

[Fig. 1] Fig. 1 is a graph of particle size distribution of the liquid droplets in the drug-containing emulsion A, measured in Example 1 of the present invention.

[Fig. 2] Fig. 2 is a graph of particle size distribution of the particulate material in the drug-containing emulsion A after removal of chloroform, measured in Example 1 of the present invention.

[Fig. 3] Fig. 3 is a graph of particle size distribution of the particulate material in the supernatant of the drug-containing emulsion A after removal of chloroform, measured in Example 1 of the present invention.

[Fig. 4] Fig. 4 is a photograph to be used as substitute of drawing, which was prepared by subjecting the supernatant obtained after centrifugation of the drug-containing emulsion A obtained in Example 1 of the present invention to negative staining with uranyl acetate, followed by observation with transmission electron microscopy.

[Fig. 5] Fig. 5 is a photograph to be used as substitute of drawing, which was prepared by subjecting the supernatant obtained after centrifugation of the drug-containing emulsion A obtained in Example 2 of the present invention to embedding in ice, followed by observation with transmission electron microscopy.

### Best Modes for Carrying Out the Invention

The present invention will be explained below by referring to one embodiment. However, it is to be understood that the examples or the like shown below are by no means restrictive and any modifications can be added thereto insofar as they do not depart from the scope of the present invention.

### [I. Particulate material dispersion composite]

The particulate material dispersion composite of the present invention is a composite in which a particulate material of the present invention is dispersed in water.

### [1. Water]

In the particulate material dispersion composite of the present invention, water constitutes a system in which the particulate material of the present invention is dispersed.

No particular limitation is imposed on the state of existence of water insofar as the intent of the present invention is not significantly impaired. Therefore, water may be in a liquid state or in a solid state. To cite a concrete example, water may be in a liquid state at the time of use but may be in the solid state at the time of storage. The scope of the right of the particulate material dispersion composite of the present invention is not affected by the state of existence of water, which is a medium. The composite of either state is the particulate material dispersion composite of the present invention.

### [2. Particulate material]

The particulate material of the present invention is a particulate material which contains a poorly water-soluble specific material, a polymer and a lipid, and usually has a surfactant on its surface.

In this specification, the term poor solubility means not only that a solute is poorly soluble in a medium (in this case, water) but that a solute is totally insoluble in a medium. More concretely, the term includes such cases in which a hydrophobic substance is poorly soluble in hydrophilic solvent and hydrophilic substance is poorly soluble in hydrophobic solvent.

### [i. Specific material]

The specific material here indicates a material which is contained in the particulate material of the present invention and forms a part of the particulate material of the present invention. There is no special limitation on its kind insofar as it is a compound poorly soluble in water and the intent of the present invention is not significantly impaired. One of the advantages of the present invention is that the particle diameter of the microparticles containing this specific material (namely, particulate material of the present invention) can be made very small and they can be dispersed in water in a stable manner. Therefore, when a compound for which micronization of particle diameter and stabilization of its dispersion have been difficult by the previously known methods is used as specific material, which is a material for the particulate material, the advantage of the present invention can be displayed preferably fully. Crystalline compounds or compounds which are solid at normal temperature are particularly suited as specific material.

As examples of a property of the specific material whose micronization of the particle diameter and stable dispersion was difficult previously can be cited a property that it is easy to crystallize or it is a solid at normal temperature. According to the present invention, it is possible to disperse in a stable manner even a particulate material containing specific materials with the above property.

In this relation, when a specific material, whose dispersion is unstable as a previously prepared dispersion composite, in which the specific material is dispersed in water, is used, the above advantage can be made use of effectively. Previously, in such dispersion composite, the specific material precipitated out or aggregated with the passage of time. However, in the particulate material or particulate material dispersion composite of the present invention, this precipitation or aggregation is prevented by the presence of a polymer and lipid used in the process of production. Accordingly, it is possible to disperse such a specific material in a stable manner.

Furthermore, it is preferable that the particulate material of the present invention is poorly soluble in water. Therefore, it is preferable that each component contained in the particulate material of the present invention such as a specific material is poorly soluble in water. By the conventional art, it has been difficult to disperse particulate material which is poorly water soluble in water in a stable manner. Therefore, the above-mentioned advantage of the present invention can be displayed fully when a compound poorly soluble in water is used as particulate material of the present invention.

That the specific material is poorly soluble in water means that the specific material does not dissolve in water to the extent that allows the formation of the particulate material of the present invention in the particulate material dispersion composite of the present invention. Concretely, the solubility of the specific material in water in a liquid state, under conditions of normal temperature and normal pressure (namely, 25 °C, 1013 hPa), should be usually 100 gram/L or less, preferably 50 gram/L or less, more preferably 10 gram/L or less. There is no lower limit to the solubility, but usually it is 0 gram/L or more.

Furthermore, it is preferable that the specific material is soluble in at least one kind of solvent which is non-miscible with water (hereinafter referred to as "non-miscible solvent" as appropriate). It is also preferable that the polymer and lipid are also soluble in this non-miscible solvent in which the specific material can dissolve, because it is preferable to use, at the time of production of the particulate material of the present invention, a non-miscible solvent in which the specific material, polymer and lipid are all soluble.

Examples of the specific material contained in the particulate material include organic compounds and inorganic compounds. Particularly suitable are known drugs, inorganic compounds, pigments and dyes.

As examples of the specific material can be cited drugs, which have various physiological activities as listed below:analgesic, antiinflammatory, anthelmintic, antiarrhythmia, antibiotics (including penicillins), anticoagulant, antihypotensive, antidiabetic, anticonvulsant, antihistamine, antihypertensive, antimuscarinic, antimycobacterium, antineoplastic, immunosuppressant, antithyroid, antivirus, antianxiety (hypnotic and nerve relaxant), astringent, adrenergic β-receptor blocker, blood preparation and plasma substitute, cardiotonic, contrast media, corticosteroid, anticough (antitussive and mucolytic), diagnostic, diagnostic imaging agent, diuretic, dopaminergic (anti-Parkinson), hemostatic, immunomodulator, lipid-modifier, muscle relaxant, parasympathomimetic, parathyroid-related calcitonin, bisphosphonate, prostaglandin, radioactive drug, sex hormone (includes steroids), antiallergy, stimulant, anorectic, sympathomimetic, thyroid drug, vasodilator and xanthines.

Listed below are preferable concrete examples: 17-α-pregno-2,4-diene-20-ino-[2,3-d]-isoxazole-17-ol (danazole), 5α,17α,-1'-(methylsulfonyl)-1'Hpregno-20-ino-[3,2-c]-pyrazol-17-ol (steroid A), [6-methoxy-4-(1-methylethyl)-3-oxo-1,2-benzisothiazol-2(3H)-yl]methyl-2,6-dichlorobenzoate-1,1-dioxide, 3-amino-1,2,4-benzotriazine-1,4-dioxide, hyposulfam, hyposulfan, camptotecin, acetaminophen, acetylsalicylic acid, amiodarone, colestyramine, colestipol, chromoline sodium, albuterol, sucralfate, sulfasalazine, minoxidil, temazepam, alprazolam, propoxyphene, auranofin, erythromycin, cyclosporine, acyclovir, ganciclovir, etopocide, mefaran, methotrexate, minoxanthrone, daunorubicin, megesterol, tamoxifen, medroxyprogesterone, nystatin, terbutaline, amphotericin B, aspirin, ibuprofen, naproxen, indomethacin, diclofenac, ketoprofen, flurbiprofen, diflomisal, ethyl-3,5-diacetoamide-2,4,6-triiodobenzoate, ethyl(3,5-bis(acetylamino)-2,4,6-triiodobenzoyloxy)acetate and ethyl-2-(3,5-bis(acetylamino)-2,4,6-triiodobenzoyloxy)acetate. Particularly preferable are naproxen and indomethacin. These drugs can be used, being contained in the particulate material.

Examples of inorganic materials used as specific materials include metal such as gold, silica, titanium oxide, clay and talc. Thus, it is possible to use metal particles such as colloidal gold or inorganic microparticles, being contained in the particulate material.

Furthermore, examples of specific materials include pigments as listed below:quinacridone pigment, quinacridonequinone pigment, dioxazine pigment, phthalocyanine pigment, anthrapyrimidine pigment, anthanthrone pigment, indanethrone pigment, flavanethrone pigment, perylene pigment, diketopyrrolopyrrole pigment, perynone pigment, quinophthalone pigment, anthraquinone pigment, thioindigo pigment, metal complex pigment, azomethine pigment and azo pigment. These pigment particles can be used, being contained in the particulate material.

Furthermore, examples of specific materials include dyestuffs such as lipophilic dye, direct dye, acid dye, basic dye and azoic dye.

Other dyestuffs applicable include dyestuffs used for ordinary writing-recording liquid such as those listed below: coumarin dye, perylene dye, dicyanovinyl dye, azo dye (for example, pyridone azo dye, bis azo dye, tris azo dye, benzene azo dye, heterocyclic azo dye etc.), quinophthalone dye, aminopyrazole dye, methine dye, dicyanoimidazole dye, indoaniline dye and phthalocyanine dye. These dye particles can be used, being contained in the particulate material.

The specific material can be used either as a single one or as a mixture of more than one kind in any combination and in any ratio.

### [ii. Auxiliary component]

In addition to the specific materials mentioned above, a polymer and a lipid are contained in the particulate material of the present invention. In the following explanation, the polymer and lipid contained in the particulate material of the present invention are called "auxiliary component" collectively, as appropriate.

The particulate material of the present invention is allowed to contain a polymer and a lipid, this promoting the stability of the dispersion of the particulate material. The role of the polymer and the lipid is not clear but is considered as follows. Namely, the polymer functions as a carrier of the particulate material and works to prevent the aggregation of the specific material in the particulate material. The lipid is considered to be located in the outer part of the particulate material and contributes to enhance the stability of the dispersion of the particulate material by facilitating adsorption of surfactant.

Also in the production of the emulsion to be described later, when containing the non-miscible solvent used, they work to enhance the stability of the emulsion significantly. The role of the polymer and the lipid (namely, auxiliary components) in this connection is not clear either. It is considered that the auxiliary components help to decrease the specific gravity of the emulsion and facilitate adsorption of surfactant onto the surface of the emulsion, thus contributing to enhance the stability of the dispersion of the emulsion.

### <Polymer>

There is no special limitation on the kind of the polymer, insofar as the intent of the present invention is not significantly impaired. Usually, one which is poorly soluble in water and soluble in the non-miscible solvent is used.

That the polymer is poorly soluble in water means that the polymer does not dissolve in water to the extent that allows the formation of the particulate material of the present invention in the particulate material dispersion composite of the present invention. In more concrete terms, the solubility of the polymer in water in the liquid state, under conditions of normal temperature and normal pressure, is usually 30 weight % or lower, preferably 10 weight % or lower, more preferably 5 weight % or lower. There is no lower limit but usually, it is 0 weight % or higher.

As described above, it is preferable that the polymer is soluble in at least one kind of non-miscible solvent. From the standpoint of ease of preparation of the particulate material of the present invention, it is preferable that it is soluble in a non-miscible solvent in which the specific material and the lipid are soluble.

Furthermore, it is preferable that the polymer is phase-separated from the lipid so that it can be a carrier in the particulate material. In order to examine whether phase-separation occurs or not, the same amounts of the polymer and lipid (for example, 1 gram each) are mixed (for example, mixed in a 5 cc vial container in an arbitrary manner) and observed for occurrence of phase-separation at a temperature usual for the preparation of the particulate material dispersion composite of the present invention (for example, room temperature).

Phase-separation here indicates that non-miscible phases with an interface in-between are formed, such as solid - solid, solid - liquid and liquid - liquid. It is allowable that the polymer and the lipid are distributed in each phase at a certain ratio.

In relation to this, it is preferable that a polymer is poorly soluble in a lipid when the lipid is in the liquid state, as mentioned above. More concretely, the solubility of the polymer in the lipid, under conditions of normal temperature and normal pressure, is usually 500 gram/L or less, preferably 300 gram/L or less, more preferably 100 gram/L or less. Under these conditions, the polymer and the lipid does not mix with each other in the process of removal of the non-miscible solvent at the time of production. The polymer works as a carrier of the particulate material and the lipid promotes the adsorption of surfactant, both of them functioning effectively. The particle diameter of the particulate material of the present invention can be made smaller and stability of the particle diameter with the passage of time can be made to increase. There is no lower limit to the solubility, but usually it is 0 gram/L or more.

The state of existence of the polymer is not limited insofar as the intent of the present invention is not significantly impaired. It may be a liquid or a solid. However, it is usually preferable that the polymer is a solid from the standpoint of invariable stability of particle diameter with the passage of time.

There is no special limitation on the molecular weight of the polymer, insofar as the intent of the present invention is not significantly impaired. The weight-average molecular weight of the polymer is usually 1000 or higher, preferably 3000 or higher, more preferably 5000 or higher, and usually 1000000 or lower, preferably 700000 or lower, more preferably 500000 or lower. The reason is as follows. When the molecular weight is below the above range, it is possible that the polymer can not function as a carrier. When the upper limit is exceeded, the viscosity may be too high and stability of the emulsion formed may decrease.

The weight-average molecular weight of the polymer is determined by GPC (gel permeation chromatography). The conditions of determination such as medium used or column used are similar to those used for the GPC determination of usual polymers.

When the particulate material or particulate material dispersion composite of the present invention is used for medical purposes or the like, biodegradable polymers can be cited as an example of the polymer. Concrete examples thereof include aliphatic polyesters, poly-α-cyanoacrylic acid esters, polyamino acids and copolymers of maleic acid anhydride. Representative examples of aliphatic polyesters include a single species polymer, copolymer, or a mixture of single species polymer and/or copolymer of α-hydroxyl carboxylic acid such as glycolic acid, lactic acid, 2-hydroxy butyric acid, 2-hydroxy valeric acid, 2-hydroxy-3-methyl butyric acid, 2-hydroxy caproic acid, 2-hydroxy isocaproic acid and 2-hydroxy caprylic acid, cyclic dimer of α-hydroxyl carboxylic acid such as glycolide and lactide, hydroxyl dicarboxylic acid such as malic acid, hydroxyl tricarboxylic acid such as citric acid. As single species polymer can be cited a lactic acid polymer. As examples of copolymer can be cited a copolymer of lactic acid / glycolic acid, and a copolymer of 2-hydroxy butyric acid / glycolic acid. As a mixture of single species polymer and/or copolymer can be cited a mixture of lactic acid polymer and 2-hydroxy butyric acid / glycolic acid copolymer.

As examples of polyamino acids can be cited poly-γ-benzyl-L-glutamine acid, poly-L-alanine and poly-γ- methyl-L-glutamine acid.

As an example of copolymer of maleic acid anhydride can be cited styrene / maleic acid copolymer.

Of these, particularly preferable are aliphatic polyesters. Of aliphatic polyesters, preferable are single species polymer, copolymer of two or more kinds, or a mixture of single species polymer and/or copolymer of α-hydroxyl carboxylic acid and cyclic dimer of α-hydroxyl carboxylic acid. Particularly preferable are single species polymer, copolymer or a mixture of single species polymer and/or copolymer of α-hydroxyl carboxylic acid.

When the above α-hydroxyl carboxylic acid, cyclic dimer of α-hydroxyl carboxylic acid, hydroxyl dicarboxylic acid and hydroxyl tricarboxylic acid have an optical-activity center in the molecule, any isomer of D-, L- and DL-compound can be used.

The above aliphatic polyesters can be prepared by any known production methods (for example, production method described in Japanese Patent Laid-Open Publication No. Sho 61-28521).Type of polymerization may be random, block or graft.

Furthermore, biocompatible polymer compounds can be preferably used. Examples include polystyrene, poly meta-acrylic acid, copolymer of acrylic acid and meta-acrylic acid, polyamino acid, dextran stearate, ethyl cellulose, acetyl cellulose, nitro cellulose, copolymer of maleic acid anhydride series, copolymer of ethylene vinyl acetate series, polyvinyl acetate, polyacryl amide, polyurethane and polyethylene.

The polymer can be used either as a single one or as a mixture of more than one kind in any combination and in any ratio.

### <Lipid>

There is no special limitation on the kind of the "lipid" of the present invention, insofar as the intent of the present invention is not significantly impaired. It is a low molecular weight compound poorly soluble in water. Preferable state of existence of the lipid is liquid. Usually, the lipid has a high boiling point. The high boiling point here indicates that it is higher than that of the non-miscible solvent. More concretely, as will be described later in the method for production, it is preferable that the difference in boiling point between the lipid and non-miscible solvent is usually 1 °C or larger, preferably 3 °C or larger, more preferably 5 °C or larger.

At the time of production of the particulate material of the present invention, a lipid used is one which is soluble in a non-miscible solvent. When used in combination with the polymer, it is inferred that the lipid is localized in the outer area of the particulate material, although the structure is not clear. The use of poorly water-soluble lipid is considered to bring about higher adsorption of surfactant and lead to easier production of the particulate material and particulate material dispersion composite of the present invention. By this effect, the stability of the particulate material dispersion composite of the present invention is considered to become higher.

That the lipid is poorly soluble in water means that the lipid does not dissolve in water to the extent that the particulate material of the present invention can be formed in the particulate material dispersion composite of the present invention. More concretely, the solubility of the lipid in water in the liquid state, under the conditions of normal temperature and normal pressure, is usually 30 weight % or lower, preferably 10 weight % or lower, more preferably 5 weight % or lower. There is no lower limit but usually, it is 0 weight % or higher.

Further, as described above, it is usually preferable that the lipid exists in the outer part of the particulate material and therefore can phase-separate from the polymer. Accordingly, it is preferable to select a lipid that can phase-separate from a polymer which functions as carrier. In order to examine whether phase-separation occurs or not, a method similar to the one described above for the polymer can be adopted.

The state of existence of the lipid is not limited insofar as the intent of the present invention is not significantly impaired. It may be a liquid or a solid. Usually, it is preferable that it is a liquid under the conditions of production. It is particularly preferable that it is a liquid under normal temperature and pressure. This is because formation of the particulate material of the present invention can then be performed efficiently. More concretely, it is preferable that the melting point of the lipid is usually 100 °C or lower, preferably 65 °C or lower, more preferably 42 °C or lower. There is no lower limit of the melting point of the lipid, but usually it is - 30 °C or higher.

As described above, the particulate material and particulate material dispersion composite of the present invention are produced via a process involving removal of a non-miscible solvent. It is preferable that the lipid is not removed in this process of removing the non-miscible solvent or removed to a smaller extent than the non-miscible solvent. Usually, removal of the above non-miscible solvent is by evaporation, and therefore, it is preferable that the lipid has a higher boiling point than the non-miscible solvent in order not to be removed. Concretely, it is preferable that the boiling point of the lipid is usually 40 °C or higher, preferably 100 °C or higher, more preferably 200 °C or higher. There is no upper limit to the boiling point of the lipid but usually, it is 500 °C or lower.

As the production of the particulate material of the present invention involves the use of a non-miscible solvent, it is preferable that the lipid is soluble in at least one kind of non-miscible solvent. From the standpoint of ease of preparation of the particulate material of the present invention, it is more preferable that it is soluble in a non-miscible solvent in which the specific material and the lipid are soluble.

There is no special limitation on the molecular weight of the lipid, insofar as the intent of the present invention is not significantly impaired. The molecular weight is usually 5000 or lower, preferably 3000 or lower, more preferably 1000 or lower. This is because, when this upper limit is exceeded, the viscosity becomes high and it may be difficult to form an emulsion in the production process. The determination of molecular weight of the lipid can be done by mass spectrometry.

As examples of lipid can be cited animal oil, vegetable oil or mineral oil whose molecular weight falls within the above specified range. Concrete examples include: vegetable oil represented by soy bean oil, sesame oil, olive oil and cotton seed oil, animal oil such as tallow, cacao fat, fatty acid triglyceride, fatty acid diester of propylene glycol, alkyl ester of medium chain or higher fatty acid, alkyl ester of lactic acid, aromatic monomer, alkyl ester of dicarboxylic acid, petroleum and silicone oil. When drug products are used as a specific material of the present invention, it is preferable to use as lipid biocompatible compounds or state-authorized pharmaceutical excipients. In Japan for example, excipients listed in the Japanese Pharmaceutical Codex (edited by Japan Pharmaceutical Excipients Council) are preferable.

The lipid can be used either as a single one or as a mixture of more than one kind in any combination and in any ratio.

Furthermore, when the lipid is in the liquid state, it is preferable that the above-mentioned specific material is poorly soluble in the lipid. Accordingly, it is preferable that, as specific material contained in the particulate material of the present invention, a compound poorly soluble in the lipid is used. Concretely, the solubility of the specific material in the lipid, under conditions of normal temperature and normal pressure, should be usually 100 gram/L or less, preferably 50 gram/L or less, more preferably 10 gram/L or less. By this selection, when a medicine (drug) is used as a specific material for example, it is possible to prevent too rapid drug absorption when the medicine is absorbed in body, thus realizing effective drug release. Also, when the drug is dissolved in the lipid, drug particles may be released gradually into water and finally precipitate out. There is no lower limit to the solubility, but usually it is 0 gram/L or more.

### [iii. Other components which the particulate material of the present invention can contain]

Components other than the above mentioned specific materials, polymer and lipid may be included in the particulate material of the present invention, insofar as the advantage of the present invention is not significantly impaired. For example, lipophilic surfactant can be included in the particulate material of the present invention.

### [iv. Surfactant]

The particulate material of the present invention usually has a surfactant on its surface. The surfactant is instrumental in making the particulate material of the present invention stable in water.

There is no special limitation on the kind of surfactant and any known surfactant can be used so long as it can make the particulate material of the present invention exist in water. For example, anionic surfactant, cationic surfactant and non-ionic surfactant can be used. Also applicable as surfactant are polymers possessing surface active potential.

As examples of anionic surfactant can be cited dodecylsulfonic acid, dodecylbenzene sulfonate, decylbenzene suofonate, undecylbenzene sulfonate, tridecylbenzene sulfonate, nonylbenzene sulfonate and their sodium, potassium and ammonium salt.

As examples of cationic surfactant can be cited cetyltrimethylammonium bromide, hexadecylpyridinium chloride and hexadecyltrimethylammonium chloride.

As examples of non-ionic surfactant can be cited polyvinyl alcohol and various commercially available products such as ┌Triton┘ (X-100, X-114, X-305, N-101) of Union Carbide, ┌Tween┘ (20, 40, 60, 80, 85) of ICI, ┌Brij┘ (35, 58, 76, 98) of ICI, ┌Nonidet┘ (P-40) of Shell, ┌Igepol┘ (CO530, CO630, CO720, CO730) of Rhone Poulenc and ┌Pluronic┘ (F68, F87, F127) of Asahi Denka.

As polymers having surface active potential can be cited naturally-occurring polymer such as dextran, pectin, dextrin, chondroitin sulfate, hyaluronic acid, heparin, gum arabic, albumin, casein, gelatin and collagen, polyamino acids and synthesized artificial proteins. Also applicable as surfactant are synthesized polymers having both hydrophilic group and hydrophobic group, such as styrene-acrylic acid copolymer.

The above surfactant can be used either as a single one or as a mixture of more than one kind in any combination and in any ratio.

### [v. Physicochemical property of particulate material]

The particulate material dispersion composite of the present invention is stable and, usually, the specific material does not precipitate out even when allowed to stand, while a specific material precipitates with the passage of time from dispersion of microparticles previously known. Concretely, the particulate material dispersion composite of the present invention is stable to the extent that no precipitate is formed, namely no specific material precipitates, in the particulate material dispersion composite of the present invention when it is allowed to stand, under conditions of normal temperature and normal pressure, for usually 12 hours or longer, preferably 1 day or longer, more preferably 2 weeks or longer, still more preferably 1 month or longer.

In this connection, it is to be noted that, for the particulate material dispersion composite of the present invention, its mean diameter remains within twice or less the original diameter when it is allowed to stand for usually 12 hours or longer, preferably 1 day or longer, more preferably 2 weeks or longer, still more preferably 1 month or longer, under conditions of normal temperature and normal pressure. In other words, it is stable to the extent that the change in mean diameter of the particulate material of the present invention remains within 100 % or less of the original diameter.

The inventors of the present invention infer that the above-mentioned stability of the particulate material of the present invention is guaranteed by the presence of the polymer and lipid contained in the particulate material. Accordingly, in the particulate material of the present invention, it is so designed that the ratio of the combined weight of the polymer and lipid to the weight of the specific material is over a predetermined value.

Concretely, the ratio of the combined weight of the polymer and lipid to the weight of the specific material [{(weight of polymer) + (weight of lipid)} / weight of a specific material] is usually 1.5 or larger, preferably 2 or larger, more preferably 2.5 or larger. In this way, the particulate material of the present invention can be made stable, as described above. There is no special limitation on the upper range but it is usually 300 or smaller, preferably 100 or smaller. When this upper limit is exceeded, desirable effect of the drug may not be expected.

In order to estimate the above ratio of the combined weight of the polymer and lipid to the weight of the specific material, the amount of the specific material, polymer and lipid can be measured by, for example, NMR or liquid chromatography, and the ratio can be calculated.

It is preferable that the mean diameter of the particulate material of the present invention is usually 1 nm or larger, preferably 3 nm or larger, more preferably 5 nm or larger, and usually 1 µm or smaller, preferably 500 nm or smaller, more preferably 100 nm or smaller. When the mean particle diameter is 200 nm or smaller, as one of the preferable modes, can be cited a particulate material whose maximum diameter is 500 nm or smaller, preferably 400 nm or smaller, more preferably 250 nm or smaller. This particulate material consists of very stable particles with a very small diameter and is expected to be used in various useful ways, taking advantage of its small size in particle diameter and excellent stability in water. The small particle diameter of the particulate material of the present invention leads to its larger specific surface area and, when the particulate material or particulate material dispersion composite of the present invention is used as drug product, the effect due to the particulate material can be exhibited efficiently. When the diameter exceeds the above upper limit, the above effect may not be expected. When it is below the above lower limit, the amount of surfactant to be used necessary may be too large.

The measurement of the diameter of particles in water such as particulate material of the present invention or liquid droplet in an emulsion described later can be carried out by the dynamic light scattering method using, for example, ┌FPAR-1000┘ (Otsuka Denshi) or ┌Microtrack UPA┘ (Microtrack).The measurement can also be done by laser diffractometry, using, for example, "LA920"(Horiba Seisakusho). Electron microscopic measurement by use of scanning electron microscope (SEM) or transmission electron microscope (TEM) is also available. Of these methods, preferable is a measurement based on dynamic light scattering method.

### [3. Other constituent]

The particulate material dispersion composite of the present invention may contain additional constituents, insofar as the advantage of the present invention is not significantly impaired. Therefore, constituents other than those described above may be contained in water. No particular limitation is imposed on these additional constituents, and any known materials can be used insofar as the intent of the present invention is not significantly impaired. These additional constituents may be contained also when the particulate material of the present invention is isolated from the particulate material dispersion composite of the present invention.

Examples of constituents that may be contained in water include: chloride such as KCl and NaCl, buffer, pH adjusting agent, viscosity adjusting agent, coloring agent, preservative, fungicide, evaporation-preventing agent and perfume.

Furthermore, for example, a solvent miscible with water can also be contained in water. Concrete examples include hydrophilic solvent such as alcohol, tetrahydrofuran (THF), and methylethyl ketone (MEK).

These constituents can be contained either as a single one or as a mixture of more than one kind in any combination and in any ratio.

### [4. Ratio of each component used]

There is no special limitation on the amount of the specific material in the particulate material dispersion composite of the present invention, insofar as the intent of the present invention is not significantly impaired. It is preferable that the amount is usually 0.01 gram/L or more, preferably 0.05 gram/L or more, more preferably 0.1 gram/L or more, still more preferably 0.5 gram/L or more, and usually 500 gram/L or less, preferably 300 gram/L or less, more preferably 100 gram/L or less. The reason is as follows. When the amount is below the above range, the specific material may dissolve in water and may not be retained in droplets of the emulsion prepared in the production process. When the upper limit is exceeded, the above-mentioned emulsion may not be stable enough and the specific material may precipitate out in water.

Further, there is no special limitation on the amount of the polymer and lipid in the particulate material dispersion composite of the present invention, insofar as the intent of the present invention is not significantly impaired. It is preferable that it is usually 0.005 gram/L or more, preferably 0.01 gram/L or more, more preferably 0.1 gram/L or more, and usually 300 gram/L or less, preferably 200 gram/L or less, more preferably 100 gram/L or less. The reason is as follows. When the amount is below the above range, the expected advantage may not be obtained. When the upper limit is exceeded, the particulate material, which is a dispersed substance, may not exist in a stable manner.

Further, there is no special limitation on the amount of the surfactant in the particulate material dispersion composite of the present invention, insofar as it is above the critical micelle concentration. It is preferable that the ratio thereof is usually 0.01 weight % or higher, preferably 0.05 weight % or higher, more preferably 0.1 weight % or higher. When the ratio is below the above lower limit, the emulsion, which is prepared in the process of the production, may not be stable. There is no special limitation on the upper limit, but usually it is 30 weight % or lower.

It is preferable that the particulate material of the present invention has a core shell structure. This core shell structure is formed by phase-separation of the polymer and lipid. It is considered that, by this core shell structure, when the lipid forms a shell phase for example, adsorption of surfactant is accelerated and stability of the microparticles on storage becomes higher. Confirmation of this core shell structure can be made by embedding the particulate material of the present invention in resin, for example, cutting out a cross sectional specimen, and observing the specimen with an electron microscope such as SEM or TEM. As the specimen of 100 nm or thinner of the particulate material of the present invention is difficult to prepare, phase contrast TEM is preferable for confirmation of the structure. As phase contrast TEM, JEM-3100FFC (Nihon Denshi, reference: Journal of Electron Microscopy 54 (I): 79 - 84 (2005)) can be used.

### [II. Method for production]

The method for producing the particulate material dispersion composite of the present invention (hereinafter referred to as "the method for producing the present invention", as appropriate) comprises a step wherein a non-miscible solvent is removed from an emulsion in which liquid droplets containing the above non-miscible solvent, specific material, polymer and lipid are dispersed in water.

### [1. Preparation of emulsion]

The emulsion used in the method for producing the present invention consists of dispersion of liquid droplets in water, as described above. In the liquid droplets the above specific material, polymer, lipid and non-miscible solvent are contained. Further, surfactant is usually contained in the emulsion.

Water, specific material, polymer, lipid and surfactant are as described before.

### [i. Non-miscible solvent]

A non-miscible solvent used in the method for producing the present invention is a solvent which is non-miscible with water and in which the specific material, polymer and lipid are soluble. There is no special limitation on the kind of the non-miscible solvent insofar as the particulate material of the present invention can be obtained. Usually, organic molecules with low boiling point, namely, organic molecules with boiling point lower than that of the lipid, is used.

That the above specific material, polymer and lipid are soluble in the non-miscible solvent means that the specific material, polymer and lipid each are soluble to the extent that allows the production of the particulate material and particulate material dispersion composite of the present invention. In more concrete terms, the solubility of the respective specific material, polymer and lipid in the non-miscible solvent in the liquid state is usually 0.01 weight % or higher, preferably 0.05 weight % or higher, more preferably 0.1 weight % or higher under conditions of normal temperature and normal pressure. There is no upper limit but usually, it is 50 weight % or lower.

On the other hand, that the non-miscible solvent is non-miscible with water means that phase separation occurs between them when the non-miscible solvent and water are present together.

It is preferable that the non-miscible solvent is volatile from the standpoint of ease of its removal in its removal step described later. Therefore, the boiling point of the non-miscible solvent is preferably low. In concrete terms, the boiling point of the non-miscible solvent is usually 0 °C or higher, preferably 5 °C or higher, more preferably 10 °C or higher, and usually 180 °C or lower, preferably 150 °C or lower, more preferably 120 °C or lower.

In this connection, it is preferable that the boiling point of the non-miscible solvent is lower than that of the polymer and lipid, particularly that of lipid. Concretely, the difference between the boiling point of the non-miscible solvent and that of the polymer and lipid is usually 1 °C or larger, preferably 3 °C or larger, more preferably 5 °C or larger. There is no upper limit to this difference, but usually it is 500 °C or smaller.

Concrete examples of the non-miscible solvent include: halogenated hydrocarbon such as dichloromethane, chloroform, dichloroethane, trichloroethane and carbon tetrachloride; ether such as ethyl ether and isopropyl ether; ketone such as methylethyl ketone; fatty acid ester such as ethyl acetate and butyl acetate; aromatic hydrocarbon such as benzene, toluene and xylene; alcohol such as butanol. Of these, preferable is halogenated hydrocarbons, and chloroform is particularly preferable.

The non-miscible solvent can be used either as a single one or as a mixture of more than one kind in any combination and in any ratio.

### [ii. Composition of the emulsion]

The amounts of the specific material and surfactant in the emulsion are the same as what has been described above for the amounts in the particulate material dispersion composite of the present invention.

Further, there is no special limitation on the amounts of the polymer and lipid in the emulsion, insofar as the intent of the present invention is not significantly impaired. It is preferable that the amounts are usually 0.01 gram/L or more, preferably 0.1 gram/L or more, more preferably 1 gram/L or more, and usually 500 gram/L or less, preferably 300 gram/L or less, more preferably 100 gram/L or less. The reason is as follows. When the amount is below the above range, sufficiently stable particulate material of the present invention may not be obtained after the non-miscible solvent is removed. When the upper limit is exceeded, it is likely that the emulsion is not formed adequately.

Similarly to the case of the particulate material dispersion composite of the present invention, it is preferable that the ratio of the combined weight of the polymer and lipid to the weight of the specific material, namely [{(weight of polymer) + (weight of lipid)} / (weight of specific material)], in the emulsion, with each component contained in liquid droplets, is usually kept at 1.5 or higher, preferably 2 or higher, more preferably 2.5 or higher. The emulsion is then kept stable and therefore the particulate material dispersion composite of the present invention can be obtained securely. There is no special limitation on the upper range but it is usually 300 or smaller, preferably 100 or smaller. When this upper limit is exceeded, in the case a drug is used as a specific material for example, it is possible that the drug may not exhibit its effect fully.

There is no special limitation on the ratio of the polymer to lipid insofar as the intent of the present invention is not significantly impaired. The ratio {weight of polymer / (weight of polymer + weight of lipid)} is usually 1 weight % or higher, preferably 5 weight % or higher, more preferably 10 weight % or higher, and usually 99 weight % or lower, preferably 95 weight % or lower, more preferably 90 weight % or lower. When the ratio is below the above range, it is possible that the polymer can not function as a carrier. When the upper limit is exceeded, stability of the dispersion may decrease.

There is no special limitation on the amount of the non-miscible solvent used, insofar as the productions of the particulate material dispersion composite and particulate material of the present invention are possible. The ratio of (weight of specific material + weight of polymer + weight of lipid) / (weight of specific material + weight of polymer + weight of lipid + weight of non-miscible solvent) is preferably adjusted in such a way that it is usually 0.1 weight % or higher, preferably 1 weight % or higher, more preferably 3 weight % or higher, and usually 50 weight % or lower, preferably 45 weight % or lower, more preferably 40 weight % or lower. The reason is as follows. If the ratio is below the above lower limit, the productivity may decline. If the upper limit is exceeded, the viscosity in the emulsion will increase, which may lead to the difficulty in emulsion formation.

In relation to water, the weight ratio of liquid droplets to the weight of the whole emulsion, namely, {(weight of specific material + weight of polymer + weight of lipid + weight of non-miscible solvent) / total weight of emulsion} is usually 0.5 weight % or higher, preferably 1 weight % or higher, more preferably 5 weight % or higher, and usually 50 weight % or lower, preferably 40 weight % or lower, more preferably 20 weight % or lower. The reason is as follows. If the ratio is below the above lower limit, the productivity may decline. When the upper limit is exceeded, it is likely that the emulsion is not formed in a stable manner.

### [iii. Process of mixing]

When the emulsion is prepared, water, the specific material, the polymer, the lipid, the surfactant, the non-miscible solvent and appropriately used other components are mixed. The order of mixing can be arbitrary insofar as the above emulsion can be obtained. However, in order to obtain the above emulsion securely, it is desirable to prepare a solution of the specific material, polymer and lipid in the non-miscible solvent (solution for liquid droplets) and a solution or dispersion of the surfactant in water (solution for water), and then mix the two.

It is preferable to include a step of micronization after each component is mixed. In other words, it is preferable to micronize the liquid droplets in the emulsion and reduce the particle diameter of the liquid droplets. This will facilitate the formation of the emulsion by micronizing the diameter of the liquid droplets.

There is no special limitation on the concrete method of micronizing the liquid droplets, and any known method such as stirring can be applied insofar as it facilitates the formation of the emulsion. For example, it is desirable to use such dispersing instruments as beads mill, roll mill, sand mill, paint shaker, ultrasonic dispersion equipment and microfluidizing equipment (Microfluidizer: registered trade mark).Of these, preferable is ultrasonic dispersion equipment. Particularly preferable is application of ultrasonic dispersion to the emulsion after mixing, which is quite effective in micronizing the liquid droplets in the emulsion.

When ultrasonic dispersion is effected, there is no special limitation on the power of the ultrasonic dispersion equipment used insofar as the intent of the present invention is not significantly impaired. Usually, it is preferable that the power is 10 W or higher and 6000 W or lower. The power outside this upper limit may be used. However, if the power is too high, it is possible that fragments of chips, used for ultrasonic dispersion, contaminate the emulsion.

No particular limitation is imposed on the frequency of ultrasonic wave when ultrasonic dispersion is effected, insofar as the micronization of the liquid droplets can be effected. Usually, it is 1 kHz or higher, preferably 5 kHz or higher, more preferably 10 kHz or higher, and usually 3 MHz or lower, preferably 100 kHz or lower, more preferably 30 kHz or lower.

No particular limitation is imposed on the length of time of ultrasonic dispersion, insofar as the intent of the present invention is not significantly impaired. It depends on the amount of the emulsion subjected to ultrasonic dispersion. Usually, it is 1 second or longer, preferably 15 minutes or longer, and usually 72 hours or shorter, preferably 1 hour or shorter.

Further, no particular limitation is imposed on the temperature condition of ultrasonic dispersion insofar as the intent of the present invention is not significantly impaired. Usually, it is desirable to perform the treatment at a temperature close to normal temperature (25 °C).However, as it is likely that the temperature rises during the treatment of ultrasonic dispersion, the process of ultrasonic dispersion is usually performed in a water bath.

Incidentally, when ultrasonic dispersion is performed in the presence of water using halogenated hydrocarbon such as chloroform, carbon tetrachloride, methylene chloride or ethylene chloride as non-miscible solvent, hydrogen halide such as HC1 may be produced, leading to lowering of pH of the water of the emulsion. When the pH of the water is lowered, it is possible that the molecular structure of the compounds which form the particulate material, such as a specific material, changes or the surfactant does not function properly. Therefore, when micronization is effected by ultrasonic dispersion, it is preferable that buffer, pH adjusting agent or ion exchange resin is added to the water before or during ultrasonic dispersion. By this procedure, the above-mentioned lowering of pH of the water can be prevented.

There is no special limitation on the buffer, pH adjusting agent or ion exchange resin used to prevent the lowering of the pH, insofar as the particulate material dispersion composite of the present invention can be produced. Concrete examples of buffer include phosphate, carbonate, Tris {2-Amino-2-hydroxymethyl-1,3-propanediol, Tris (hydroxymethyl) aminomethane} and HEPES {2-[4-(2-hydroxyethyl)-1-piperazinyl] ethanesulfonic acid}.Examples of pH adjusting agent are alkaline substances such as NaOH.

No particular limitation is imposed on the pH of the water in the emulsion, insofar as the particulate material and particulate material dispersion composite of the present invention can be produced. From the standpoint of preventing the change of molecular structure of the compound constituting the particulate material, as described above, and preventing the malfunctioning of the polymer as carrier, it is preferable that the pH of the water is usually 1.0 or higher, preferably 1.5 or higher, more preferably 2.0 or higher, and usually 12 or lower, preferably 11 or lower, more preferably 10 or lower.

There is no special limitation on the diameter of the liquid droplets of the emulsion obtained insofar as the intent of the present invention is not significantly impaired. It is preferable that the mean diameter is usually 10 nm or larger, preferably 30 nm or larger, more preferably 50 nm or larger, and usually 200 µm or smaller, preferably 100 µm or smaller, more preferably 50 µm or smaller. The reason is as follows. When the diameter is below the above lower limit, the specific surface area of the emulsion becomes too large, possibly necessitating too much surfactant. When the upper limit is exceeded, the stability of the emulsion may decrease. Furthermore, by making the diameter of the emulsion small, its specific surface area can be made large, which makes possible the efficient production of the particulate material of the present invention advantageously. The measurement of the mean diameter of the above liquid droplet can be done by the dynamic light scattering method.

### [2. Removal of non-miscible solvent]

After preparation of the emulsion, the non-miscible solvent is removed from that emulsion. In more detail, the non-miscible solvent contained in the liquid droplets of the emulsion is removed.

There is no special limitation on the method of removal of the non-miscible solvent and any known method can be applied. Usually, the removal of the non-miscible solvent is done by in-liquid drying method.

When the non-miscible solvent is removed by in-liquid drying, there is no special limitation on the conditions of drying such as temperature, pressure or length of drying time, and any condition is allowed insofar as the removal of the non-miscible solvent is possible. To cite a suitable drying condition, the temperature is usually 200 °C or lower, preferably 150 °C or lower, more preferably 100 °C or lower. If the upper limit is exceeded, the compounds in the emulsion may decompose. There is no special limitation on the lower limit of the temperature condition, but usually, it is -50 °C or higher. The pressure is usually 100 kPa or lower, preferably 70 kPa or lower, more preferably 50 kPa or lower. No particular limitation exists on the lower end of the pressure condition, but usually, it is 1.0 x 10⁻⁶ Pa or higher. The drying time is usually 1 minute or longer, preferably 5 minutes or longer, more preferably 10 minutes or longer. No upper limit of the drying time exists but it is usually 1 week or shorter. When water is dried together with the non-miscible solvent, water may be added to the emulsion, as appropriate, during the drying.

Further, in order to accelerate the removal of the non-miscible solvent, it is preferable to carry out the drying using an instrument for drying or the like. For example, the non-miscible solvent can be evaporated off under normal pressure or under gradually reduced pressure using a propeller-type stirrer or magnetic stirrer, or it can be evaporated off using a rotary evaporator while adjusting the level of vacuum. Freeze drying can also be used. Of these methods, preferable is the use of a rotary evaporator.

### [3. Separation of particulate material]

Particles other than the particulate material of the present invention may be removed from the particulate material dispersion composite of the present invention, as appropriate. There is no special limitation on the method of this removal. One example is to conduct a step in which particles having a diameter larger than a predetermined value are removed from the water of the particulate material dispersion composite of the present invention. As the predetermined value of diameter can be adopted, for example, 1 µm. Particles in the water other than the particulate materials of the present invention usually have a diameter larger than that of the particulate materials of the present invention. Therefore, if particles having a diameter larger than that of the particulate material of the present invention are removed, it means that the particulate material of the present invention can be separated.

As concrete methods of removing the particles having a diameter larger than a predetermined value from the particulate material dispersion composite of the present invention, as described above, can be cited, for example, centrifugation, ultrafiltration, gel filtration or dialysis. It is possible to remove particles having a diameter larger than a predetermined value by these methods.

Further, if water is removed from the composite obtained, namely composite in which the particulate material of the present invention is dispersed in water, it is possible to separate the particulate material of the present invention. For example, particles having a diameter larger than a predetermined value are precipitated by centrifugation, and the supernatant is subjected to freeze drying or reduced-pressure drying. Water is then removed and the particulate material of the present invention can be obtained.

Otherwise, salting out can be used to precipitate by aggregation particles having a diameter larger than a predetermined value. Water is then removed from the supernatant by drying, leaving the particulate material of the present invention.

Therefore, if water is removed from the particulate material dispersion composite of the present invention, it is possible to obtain a particulate material containing a specific material poorly soluble in water, a polymer and a lipid, wherein the mean diameter of the particulate material is 1 µm or less, and the ratio of the combined weight of the polymer and the lipid to the weight of the specific material is 1.5 or larger.

### [4. Yield]

According to the method for producing the present invention, usually 1 % or more, preferably 3 % or more, more preferably 10 % or more of the specific material contained in the above emulsion can be obtained in a manner that it is transferred to and contained in the particulate material of the present invention. The upper limit of the yield is ideally 100 %. The yield can be analyzed, for example, by liquid chromatography after separation of the particulate material from water.

### [III. Application field]

The particulate material and particulate material dispersion composite of the present invention can be used in various industry areas, taking advantage of their small particle diameter and excellent stability. For example, when a particulate material containing a poorly water-soluble specific material is to be formed, it is possible to obtain the particulate material of the present invention as particulate material poorly soluble in water and having surfactant on the surface. If a drug, for example, is contained as a specific material of the particulate material, that drug can be dispersed in water in a stable manner and in a micronized state, even if the drug is poorly soluble in water. It is therefore possible to administer the drug appropriately as a dispersion liquid, in which the drug is dispersed in water, to the body requiring the drug.

When the particulate material and the particulate material dispersion composite of the present invention are used in medical field, it is preferable that each component of the particulate material and the particulate material dispersion composite of the present invention such as water, specific material, surfactant, polymer, lipid and other components is one approved for medical use. When a drug is used as a specific material contained in the particulate material, it is possible to use the particulate material and the particulate material dispersion composite of the present invention as excipients for medical use.

As described above, when a drug is used as specific material, the particulate material and particulate material dispersion composite of the present invention will be expected to facilitate drug absorption into the body because of their increased specific surface area, and enhance stability of the dispersion because of their micronization. Drug delivery and storage will be advantageous thanks to these properties. Furthermore, application of the method for producing the present invention to producing poorly water-soluble drugs provides a general method of micronizing drugs without requiring a large amount of energy and without being influenced by special characteristics of drugs significantly.

Furthermore, one of the advantages of the method for producing the present invention is that it makes possible easy preparation of a particulate material with a very small particle diameter. Therefore, when the particulate material of the present invention is prepared using as specific material pigments or dyes for which micronization of particles is desired, like tonner of image forming device, it is possible to obtain easily a particulate material with good quality and with a very small particle diameter.

Furthermore, the particulate material and the particulate material dispersion composite of the present invention can be used in various areas in addition to the field mentioned above. For example, when pigments or dyestuffs are used as specific materials, they can be applied to ink for ink jet printer, tonner, resist for color filter and other ink or paint.

### [Example]

The present invention will be explained more concretely below by referring to Examples. However, the present invention is not limited to these Examples and any modification can be added thereto insofar as it does not depart from the scope of the present invention.

### [Example 1]

### (1) Preparation of emulsion

Amounts of 0.03 g of s-naproxen ((s)-(+)-6-Methoxy-α-methyl-2-naphthaleneacetic acid: Aldrich), which is a drug (specific material), 0.15 g of soy bean oil (Wako Pure Drug), which is a lipid, and 0.3 g of poly-L-lactic acid (molecular weight 10000: Nakarai Tesque), which is a polymer, were dissolved in 2.52 g of non-miscible solvent chloroform (Junsei Chemical). The solution was added to a 50 mL vial container containing 0.3 g of SDS (sodium lauryl sulfate (surfactant): Nakarai Tesque) and 26.7 g of desalted water (water), and the mixture was dispersed using an ultrasonic dispersion equipment (ULTRA SONIC HOMOGENIZER UH-600S: STM). The ultrasonic dispersion equipment was adjusted so that its output chip was7 φ, output level was 5, interval level of ultrasonic irradiation was 50 %, and irradiation time of ultrasonic wave was set at 15 minutes. During the irradiation, the vial container was immersed in a water bath in order to prevent the rise in temperature. Thus, the drug-containing emulsion A was obtained as white turbid emulsion. In this drug-containing emulsion A the value of {weight of polymer + weight of lipid} / {weight of specific material} was 15.

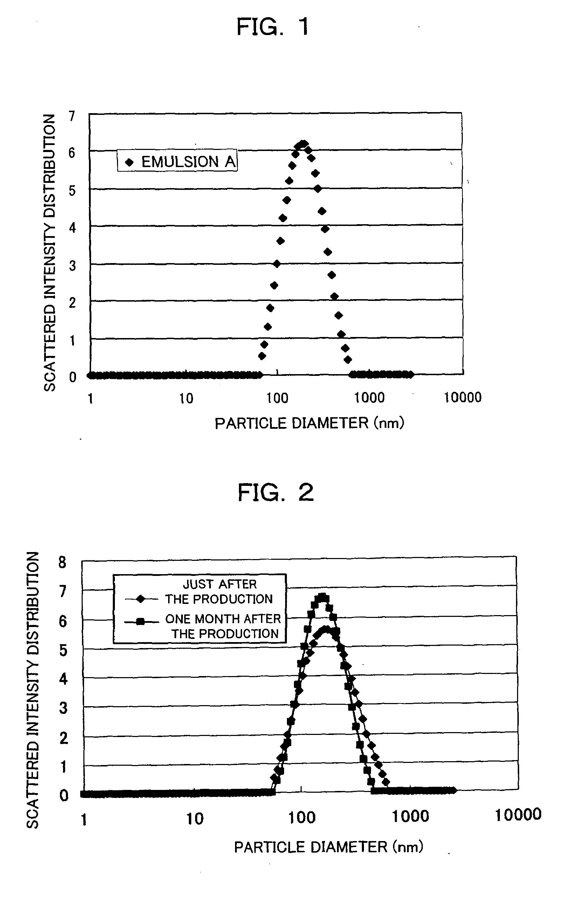

Particle size distribution of the drug-containing emulsion A was measured by a particle size distribution meter FPAR-1000 (Otsuka Denshi, probe for high concentration used). Analysis of particle size distribution was made by the Contin method using a program attached to FPAR-1000 (the same method was applied to the analyses of particle size distribution in other Examples, too). The result is shown in Fig. 1. The mean diameter of the liquid droplets in drug-containing emulsion A was estimated to be about 180 nm.

### (2) Removal of chloroform from the emulsion

In order to remove chloroform contained in the liquid droplets of the drug-containing emulsion A, 20 g of the drug-containing emulsion A was placed in a round-bottomed flask and the flask was immersed in a thermostat bath, maintained at 60 °C. The chloroform was evaporated under carefully controlled reduced pressure, using a rotary evaporator, while care was taken to avoid bumping, until the weight of the emulsion A decreased by more than the weight of the chloroform contained (1.68 g) in the liquid droplets of drug-containing emulsion A and the odor of the chloroform was no longer perceptible. By this procedure, the chloroform was removed from the liquid droplets of the drug-containing emulsion A and the chloroform-removed drug-containing emulsion A (particulate material dispersion composite), in which the particulate material was dispersed in water, was obtained as white turbid emulsion. The chloroform-removed drug-containing emulsion A obtained weighted 18.09 g.

The particle size distribution of the particulate material in the chloroform-removed drug-containing emulsion A was measured using FPAR-1000. The result is shown in Fig. 2. This result shows that the mean diameter of the particulate material in chloroform-removed drug-containing emulsion A was found to be about 150 nm.

Similar measurement was repeated one month after removal of chloroform, the result of which is also shown in Fig. 2. This result shows that the mean diameter of the particulate material in chloroform-removed drug-containing emulsion A was about 150 nm, demonstrating that little change occurred during storage.

From the above data, it was confirmed that the particulate material in the chloroform-removed drug-containing emulsion A is very stable.

Furthermore, the chloroform-removed drug-containing emulsion A was centrifuged at 10000 rpm of rotation rate and for 30 minutes of rotation time. Coarse particles then precipitated and supernatant containing fine particles was obtained.

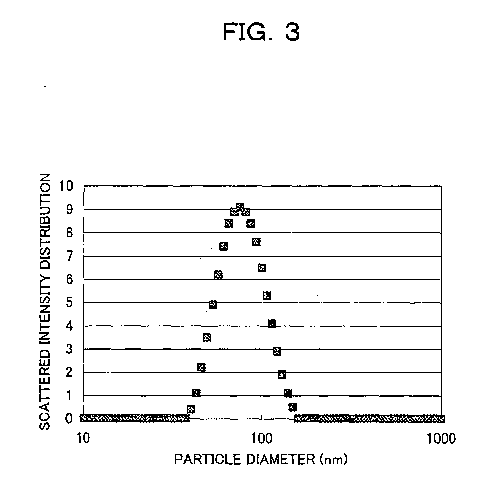

This supernatant was subjected to particle size distribution analysis of the particulate material using FPAR-1000. The result is shown in Fig. 3. The result shows that the mean diameter of the particles in the supernatant (particulate material of the present invention) is about 75 nm and particles of 250 nm or larger do not exist.

Further, the solvent of 100 µL of this supernatant was removed, 0.5 mL of ethanol was added to the residue and the ethanol solution was analyzed by liquid chromatography (column TSKgel superODS). The concentration of s-naproxen, which is the drug, was estimated to be 0.890 mg/mL. Further, 200 µL of the supernatant, after centrifugation, was ultrafiltered using Microkon YM-10 (Amicon) at 7000 rpm for 90 minutes. The drug concentration in the sample of ultrafiltrate was 0.116 mg/mL. The molecular cut-off value of this Microkon YM-10 used for this ultrafilter is 10000 (protein equivalent) and its micro-hole is considered to be 5 to 10 nm in diameter. Therefore, it is suggested that most of naproxen remained in the particulate material without being solubilized.

Furthermore, 1 mL of the supernatant was freeze-dried, the residue was all dissolved in 2 mL of methanol / chloroform (volume ratio 1:1) and analyzed by liquid chromatography (column TSKgel superODS).The amount of poly-L-lactic acid and soybean oil was found to be 7.8 mg/mL and 0.99 mg/mL, respectively. Therefore, the ratio of the combined weight of polymer (poly-L-lactic acid) and lipid (soybean oil) to the weight of the specific material (s-naproxen) was estimated to be 9.9.

When the above supernatant was allowed to stand under conditions of normal temperature and normal pressure, no precipitation of substances was observed even after 1 month and the particulate material of the particulate material dispersion composite of the present invention proved to be very stable.

### (3) Electron microscopic observation

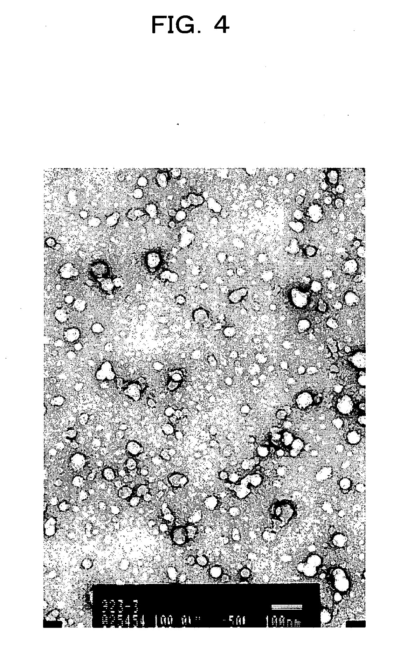

The above-mentioned supernatant after centrifugation of the drug-containing emulsion A was examined by transmission electron microscopy (TEM) after negative staining with uranyl acetate. The photograph of the observed image, to be used as substitute of drawing, is shown in Fig. 4. In Fig. 4, particulate materials of 10 to 50 nm diameter, which seem to be the particulate material of the present invention, are observed.

### [Example 2]

A drug-containing emulsion was prepared in the same manner as described for Example 1, except that the drug used was indomethacin (Sigma) and ultrasonic dispersion was performed for 30 minutes. This emulsion is called drug-containing emulsion B. The mean diameter of this drug-containing emulsion B was measured by the same method as described for Example 1. The mean diameter was found to be about 140 nm.

Further, chloroform was removed from the drug-containing emulsion B in the same manner as described for Example 1, and the mean diameter of this chloroform-removed drug-containing emulsion B (particulate material dispersion composite) was measured by the same method as described for Example 1. The diameter was found to be about 96 nm.

Furthermore, the chloroform-removed drug-containing emulsion B was centrifuged in the same manner as described for Example 1. Most of relatively large particles having a particle diameter over a predetermined value precipitated and the supernatant became almost transparent.

This supernatant was subjected to particle size distribution analysis of the particulate material using FPAR-1000. The result shows that the mean diameter of the particulate material in the supernatant (particulate material of the present invention) is about 69 nm and particles of 250 nm or larger do not exist.

Furthermore, the composition of the supernatant was analyzed by liquid chromatography in the same manner as described for Example 1. The amount of indomethacin was found to be 0.835 mg/mL and that of poly-L-lactic acid and soybean oil was found to be 11.8 mg/mL and 2.0 mg/mL, respectively. Therefore, the ratio of the combined weight of polymer (poly-L-lactic acid) and lipid (soybean oil) to the weight of the specific material (indomethacin) was estimated to be 16.5.

The amount of indomethacin contained in the filtrate after ultrafiltration of indomethacin-containing particulate material was measured in the same manner as described in Example 1 for s-naproxen in the filtrate. It was found that 0.229 mg/mL of indomethacin was contained in the filtrate after ultrafiltration. Therefore, it is suggested that most of indomethacin remained in the particulate material without being solubilized.

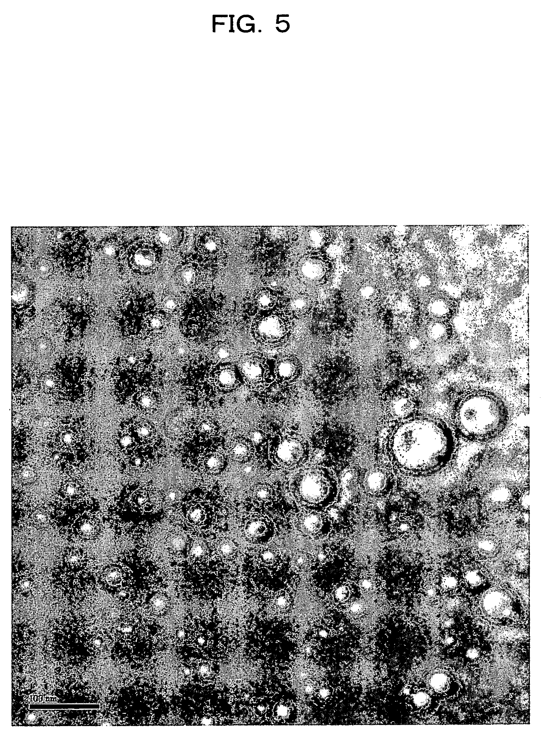

Further, in order to confirm the inner structure of the particulate material, the supernatant obtained above was frozen using an instantaneous freezing equipment EM CPC (LEICA), a specimen was prepared by ice embedding, and the specimen was observed by phase contrast TEM (JEM-3100FFC). The result is shown in Fig. 5. The particulate material of the present invention was confirmed to have a core shell structure (Refer to Fig. 5. Note that in the Fig. the scale is in 100 nm).

### [Comparative example 1]

A drug-containing e-mulsion was prepared in the same manner as described for Example 1, except that soy bean oil and poly-L-lactic acid were not used and the amount of chloroform used was 2.97 g. The drug-containing emulsion obtained was analyzed by FPAR-1000 and the mean diameter was found to be 600 nm. This drug-containing emulsion separated into white precipitate and supernatant after 2 hours.

Further, chloroform was removed immediately after preparation of the drug-containing emulsion, similarly to Example 1. The emulsion after removal of chloroform was colorless and transparent, but many minute crystals, observed visually, separated out and precipitated about 1 hour later.

### [Comparative example 2]

A drug-containing emulsion was prepared in the same manner as described for Comparative Example 1, except that the drug used was indomethacin. The drug-containing emulsion obtained separated into white precipitate and supernatant after about 20 minutes and, therefore, particle size distribution could not be measured.

Further, chloroform was removed immediately after preparation of the drug-containing emulsion, similarly to Example 1. The emulsion after removal of chloroform was colorless and transparent, but many minute crystals, observed visually, separated out and precipitated about 1 hour later.

### [Comparative example 3]

A drug-containing emulsion was prepared in the same manner as described for Example 1, except that the amount of soybean oil and poly-L-lactic acid was 0.006 g and 0.03 g, respectively, and the amount of chloroform was 2.934 g. The emulsion separated into white precipitate and supernatant after about 2 hours. The initial ratio of the combined weight of polymer (poly-L-lactic acid) and lipid (soybean oil) to the weight of the specific material (s-naproxen) was 1.2.

### [Conclusion]

From the comparison between Examples 1 and 2 and Comparative Examples 1 to 3, it has been confirmed that the particulate material in the chloroform-removed drug-containing emulsion A and B of Example 1 and Example 2, which is the particulate material dispersion composite of the present invention, is very small in particle diameter and very stable in comparison with that of Comparative Examples 1 to 3.

### Industrial Applicability

The present invention can be applied widely in various fields of industry. For example, it can be suitably applied for medical purposes, staining, drug product, ink and paint.

The present invention has been explained in detail by referring to specific embodiments. However, it is obvious for those skilled in the art that various modifications can be added thereto without departing from the intention and the scope of the present invention.

The present application is based on Japanese Patent Application (Patent Application No. 2005-190350) filed on June 29, 2005 and their entireties are incorporated by reference.

## Claims

1. A particulate material dispersion composite, wherein
a particulate material containing a specific material which is poorly soluble in water, a polymer and a lipid is dispersed in water,
the mean diameter of said particulate material is 1 µm or less, and
the ratio of the combined weight of said polymer and said lipid to the weight of said specific material is 1.5 or larger.

2. A particulate material dispersion composite, wherein
a particulate material containing a specific material which is poorly soluble in water, a polymer and a lipid is dispersed in water,
the mean diameter of said particulate material is 1 µm or less, and
said particulate material is stable even when left for 12 hours under the conditions of 25 °C and 1013 hPa.

3. A particulate material dispersion composite as defined in claim 1 or 2, wherein said particulate material has a surfactant on its surface.

4. A particulate material dispersion composite as defined in any one of claims 1 to 3, wherein said polymer is poorly soluble in water.

5. A particulate material dispersion composite as defined in any one of claims 1 to 4, wherein said specific material is poorly soluble in said lipid.

6. A particulate material dispersion composite as defined in any one of claims 1 to 5, wherein said lipid and said polymer are phase-separated from each other.

7. A particulate material dispersion composite as defined in any one of claims 1 to 6, wherein said specific material, said polymer and said lipid are all soluble in at least one kind of non-miscible solvent which is non-miscible with water.

8. A particulate material dispersion composite as defined in any one of claims 1 to 7, wherein said specific material contains a drug.

9. A method for producing the particulate material dispersion composite as defined in any one of claims 1 to 8, comprising the step of:
removing a non-miscible solvent, which is non-miscible with water, from emulsion in which liquid droplets are dispersed in water, said liquid droplets containing said non-miscible solvent, said specific material, said polymer and said lipid.

10. A method for producing a particulate material dispersion composite, comprising the steps of:
removing a non-miscible solvent, which is non-miscible with water, from emulsion in which liquid droplets are dispersed in water, said liquid droplets containing said non-miscible solvent, a specific material, a polymer and a lipid, and
removing particles having a diameter larger than a predetermined value from the water.

11. A method for producing a particulate material dispersion composite, comprising the step of :
removing a non-miscible solvent, which is non-miscible with water, from emulsion in which liquid droplets are dispersed in water, said liquid droplets containing said non-miscible solvent, a specific material, a polymer and a lipid and having 1.5 or larger value of the ratio of the combined weight of said polymer and said lipid to the weight of said specific material.

12. A method for producing the particulate material dispersion composite as defined in any one of claims 9 to 11, further comprising the step of:
micronizing said liquid droplets in said emulsion.

13. A particulate material containing a specific material poorly soluble in water, a polymer and a lipid, wherein
the mean diameter of said particulate material is 1 µm or less, and
the ratio of the combined weight of said polymer and said lipid to the weight of said specific material is 1.5 or larger.

14. A drug product containing the particulate material dispersion composite as defined in claim 8.
